# EUROPEAN PATENT APPLICATION

(11) **EP 4 778 501 A1**
(43) Date of publication of application: **22.07.2026**
(21) Application number: 26151759.3
(22) Date of filing: 14.01.2026
(51) Int. Cl.: A61G 7/018, A61B 5/11, G16H 40/63

(54) **SYSTEMS AND METHODS FOR AUTOMATED RESET OF BED SETTINGS TO PRESET RISK PROTOCOLS**

(30) Priority: 16.01.2025 US 202563746062 P
(71) Applicant: Hill-Rom Services, Inc., Batesville, IN 47006 (US)
(72) Inventor: SHIRLEY, Daniel Chase, Batesville, 47006 (US); HETTIG, Mark F., Batesville, 47006 (US); DAVIDSON, Frederick Collin, Batesville, 47006 (US); AYERS, Brandon M., Batesville, 47006 (US); CHANDRAMOULI, Ashwin, Batesville, 47006 (US); GRESKOVICH, James A., Batesville, 47006 (US); MCCOON, Keilah T., Batesville, 47006 (US)
(74) Representative: Reddie & Grose LLP

(57) **Abstract**

A bed control system for controlling settings of a bed includes a bed controller that is configured to control multiple actuators of the bed. The actuators are configured to move the bed based on instructions from the bed controller. A master computing device is remote from the bed and communicatively coupled to the bed controller. The master computing device includes a memory storing a preset risk protocol and logic that, when implemented by a processor, sends adjustment instructions to the bed controller based on a comparison between multiple current bed settings of the bed and the preset risk protocol.

## Description

The present specification generally relates to beds and, in particular, systems and methods for automated resetting of bed settings to preset risk protocols.

### TECHNICAL BACKGROUND

Many beds (e.g., hospital beds) are positionable to a configuration having the sleeping surface of the bed at a predetermined height above the floor and having side rails for inhibiting the movement of a person lying on the sleeping surface past sides of the sleeping surface and off of the bed. The sleeping surfaces of many beds can be lowered to reduce the distance between the sleeping surface and the floor, and the sleeping surfaces of such beds can often be manipulated to adjust the position of the person on the sleeping surface.

Each year, thousands of subjects fall in hospitals. Some of these falls are related to beds. The beds may have control features that allow for setting of various bed features, some of which may aid in reducing subject fall risks. Accordingly, what is needed are bed control systems and methods that can be used to reduce subject risk.

According to one aspect, a bed control system for controlling settings of a bed includes a bed controller that is configured to control multiple actuators of the bed. The actuators are configured to move the bed based on instructions from the bed controller. A master computing device is remote from the bed and communicatively coupled to the bed controller. The master computing device includes a memory storing a preset risk protocol and logic that, when implemented by a processor, sends adjustment instructions to the bed controller based on a comparison between multiple current bed settings of the bed and the preset risk protocol.

According to another aspect, a method for controlling settings of a bed using a bed control system is provided. The method includes sending current bed settings of the bed to a master computing device that is remote from the bed using a bed controller of the bed. The bed controller is configured to control multiple actuators of the bed. The actuators are configured to move the bed based on adjustment instructions from the bed controller. The current bed settings are compared to a preset risk protocol saved in memory using the master computing device and sending adjustment instructions from the master computing device to the bed controller based on the comparison thereby causing the bed controller to adjust the multiple actuators of the bed.

According to another aspect, a method for controlling settings of a bed using a bed control system is provided. The method includes entering a Falls Protocol into memory of a master computing device that is remote from the bed. Multiple current bed settings of the bed are sent to the master computing device using a bed controller of the bed. The bed controller is configured to control Head of Bed Angle, Bed Braking, Bed Height, Bed Rails Position and Bed Exit On/Off based on instructions from the bed controller. The master computing device compares the multiple current bed settings to the Falls Protocol and sends adjustment instructions to the bed controller based on the comparison thereby causing the bed controller to adjust the bed based on the step of comparing.

The invention will now be further described with reference to the accomapnying drawings in which:
FIG. 1 is a side perspective view of a bed including a subject control unit, according to one or more embodiments shown and described herein;
FIG. 2 is a front view of the subject control unit of FIG. 1 in isolation, according to one or more embodiments shown and described herein;
FIG. 3 is a schematic view of a bed control system for controlling operation of the bed of FIG. 1, according to one or more embodiments shown and described herein;
FIG. 4 is a schematic view of a user input device for use in entering information for use by the bed control system of FIG. 3, according to one or more embodiments shown and described herein; and
FIG. 5 illustrates a method of controlling the bed of FIG. 1, according to one or more embodiments shown and described herein.

A subject fall may be defined as an unplanned descent to the floor with or without injury to the subject. A fall may result in increased health care utilization. Some of these falls are related to bed usage. In this regard, hospitals may have a number of risk protocols for their beds that hospital personnel believe can reduce issues related to bed usage. The risk protocols can be based on a number of potential health issues and risks, such as falls risk, pulmonary risk and skin risk. For example, a bed "Falls Protocol" refers to a predetermined set of fall prevention measures to reduce occurrences of subjects falling from beds. The Falls Protocol can differ from hospital-to-hospital and even from subject-to-subject, depending on the circumstances of the particular subject. Some bed Falls Protocol and other risk protocol measures can relate to Head of Bed (HOB) Angle, Bed Braking, Bed Height, Bed Rails Position and Bed Exit On/Off.

The systems and methods described herein can be used to monitor various current bed features, compare them to the preset risk protocol for the hospital and/or subject, for example, depending on whether the subject is at risk for falls or not, a pulmonary risk and/or a skin risk, and change the current bed settings to meet the preset risk protocol. This information regarding risk can be obtained from a database and/or from an assessment at admission of the subject, which can be added to the database, for example, using an application. When the subject is admitted, a notification may be automatically sent to a caregiver and the preset risk protocol may be displayed on a display that is accessible to the caregiver. For a fall risk, as an example, before the subject is placed in the bed, the bed may be zeroed so that the subject can be accurately weighed. The caregiver may check that there is nothing except pre-approved linens on the bed during the zeroing process. The Falls Protocol may include setting a bed exit alarm On. In this regard, a bed exit sensitivity may be set depending on the needs of a particular subject and/or the Falls Protocol. In some embodiments, the bed may have three sensitivity options, most sensitive, moderately sensitive and least sensitive, which determine how the bed alarms will react to in-bed movement. The bed exit alarm may not be armed until the subject is in the bed and a light on the bed may illuminate. For a falls risk subject, there are a number of measures that may also occur automatically before the subject enters the bed. As examples, the bed height may be set to the lowest setting to aid in entering the bed and the number of raised side rails may be reduced, such as all of the side rails being placed in a lowered position. Further, the brakes may be activated to inhibit movement of the bed while onboarding. A Head-of-Bed angle may be set to a preselected angle to horizontal, such as 30 degrees.

The present description is directed generally to systems and methods of controlling the settings of a bed automatically and in a straightforward fashion. The bed control system can include the bed with a bed controller that can control multiple actuators of the bed in order to adjust various settings of the bed automatically. The actuators move the bed based on instructions from the bed controller which receives instructions from a master computing device remote from the bed and communicatively coupled to the bed controller. The master computing device includes a memory including a preset risk protocol and logic that, when implemented by a processor, sends adjustment instructions to the bed controller based on a comparison between current bed settings of the bed and the preset risk protocol.

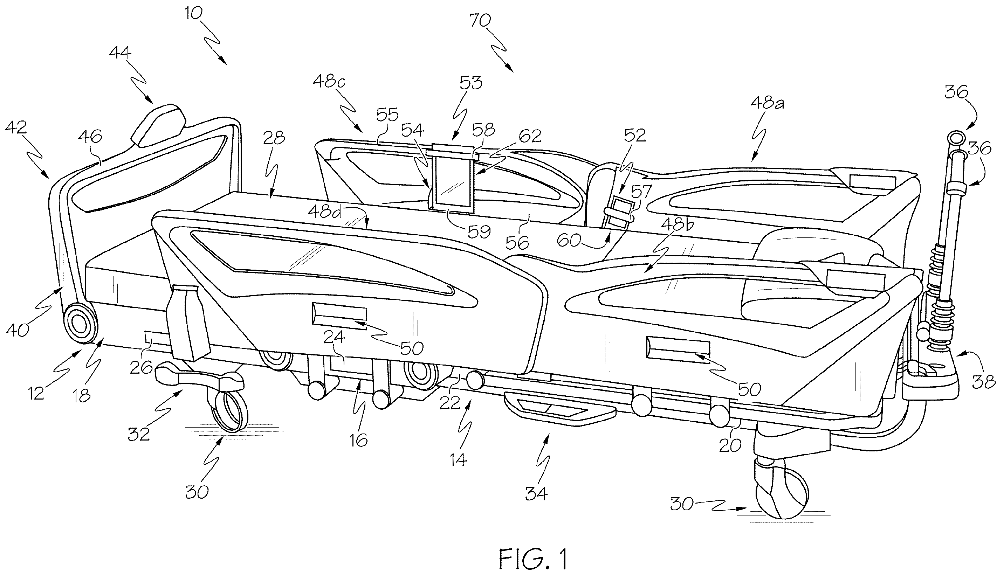

Referring to FIG. 1, an illustrative bed 10 includes a frame 12 that, in turn, includes a base frame 14, an upper frame 16, and a subject support deck 18. The subject support deck 18 includes a head section 20, a seat section 22, a thigh section 24, and a foot section 26. The bed 10 has a mattress 28, sometimes referred to as a subject support surface, supported atop sections 20, 22, 24, 26. One or more of sections 20, 22, 24, 26 are selectively movable relative to upper frame 16 to support a subject in a variety of positions. The bed 10 includes various actuators that move the movable sections 20, 22, 24, 26 and that raise, lower, and tilt upper frame 16 relative to base frame 14.

The bed 10 also may include casters 30 and a pair of butterfly brake pedals 32 which are used to brake and release casters 30 via a braking linkage or other similar mechanism. The braking linkage may be actuated manually using a caregiver's foot and/or may be automatically actuated using one of the actuators noted above. Another foot pedal assembly 34 may be provided on the base frame 14 of illustrative bed 10 for raising and lowering one portion of the bed relative to another. A pair of push handles 36 are provided at a head end 38 of bed 10 and are used by caregivers to push bed 10 from one location to another when casters 30 are released.

The bed 10 may have a footboard 40 coupled to support deck section 18 at a foot end 42 of bed 10. In the illustrative example, a caregiver control pod 44 extends upwardly from a central region of a top bar 46 of footboard 40. Caregiver controls, in the form of user inputs such as one or more buttons, switches, touchscreen displays, and the like may be provided on the surface of control pod 44 that faces away from mattress 28. Thus, the caregiver controls are generally inaccessible to a subject lying on mattress 28.

The bed 10 also includes a set of side rails 48a, 48b, 48c, 48d as shown in FIG. 1. In the illustrative example, side rails 48a, 48b are head end side rails (aka "head rails") and side rails 48c, 48d are foot end side rails (aka "foot rails"). Each of side rails 48a, 48b, 48c, 48d has a hand-receiving recess 50 through which a release handle is accessible for releasing a locking mechanism so that the respective side rail 48a, 48b, 48c, 48d can be moved from a respective raised position (shown in FIG. 1) to a respective lowered position. In some embodiments, the side rails 48a, 48b, 48c, 48d may be actuated automatically between raised and lowered positions using one or more actuators noted above.

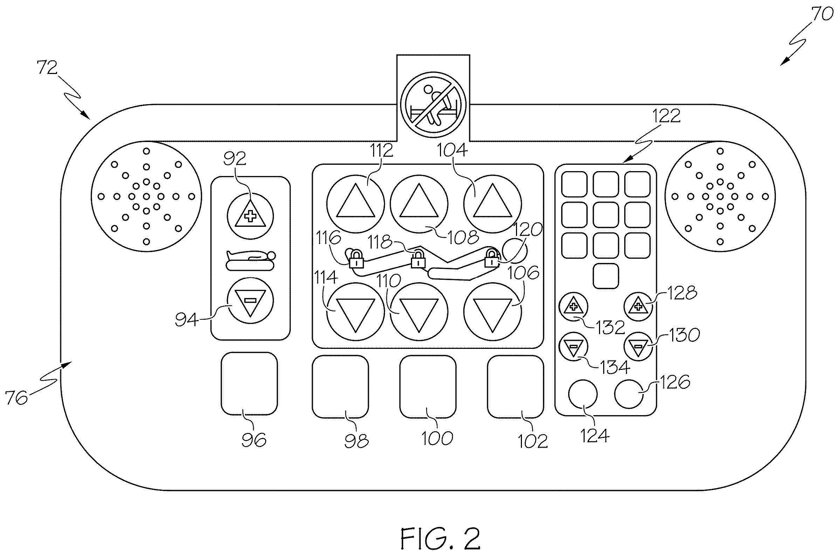

Referring to FIG. 2, the bed 10 may also include a subject control unit 70 that includes a housing 72. The subject control unit 70 may be mounted to the bed 10 at any suitable location that can be accessed by the subject while in the bed 10. In some embodiments, a subject control unit may not be provided.

A first side 76 of housing 72 of subject control unit 70 may have user inputs and various indicia including the following:
Mattress firmer button 92 - pressed to signal a pneumatic system of bed 10 to increase the pressure in one or more air bladders of mattress 28;
Mattress softer button 94 - pressed to signal the pneumatic system of bed 10 to decrease the pressure in one or more air bladders of mattress 28;
Subject egress button 96 -pressed when a subject is egressing from a side of mattress 28 to increase the pressure in one or more seat zone bladders of mattress 28;
Room light button 98 - pressed to turn on and turn off a room light;
Nurse call button 100 - pressed to send a signal to one or more master computing device devices, including remote master computing device devices, of a nurse call system;
Reading light button 102 - pressed to turn on and turn off a reading light;
Head up button 104 - pressed to signal a control system of bed 10 to raise the head section 20;
Head down button 106 - pressed to signal the control system of bed 10 to lower the head section 20;
Thigh up button 108 - pressed to signal the control system of bed 10 to raise the thigh section 24;
Thigh down button 110-pressed to signal the control system of bed 10 to lower the thigh section 24;
Foot up button 112 - pressed to signal the control system of bed 10 to raise the foot section 26;
Foot down button 114 - pressed to signal the control system of bed 10 to lower the foot section;
Foot section lockout indicia 116 - illuminates when the foot section 26 is locked out from movement;
Thigh section lockout indicia 118 - illuminates when the thigh section 24 is locked out from movement;
Head section lockout indicia 120 - illuminates when the head section 20 is locked out from movement;
Phone key pad 122 - includes buttons for numbers 0-9 arranged as a traditional telephone keypad for us by a subject in placing a telephone call;
Radio button 124 - pressed to turn a radio on and off;
Television button 126-pressed to turn a television on and off;
Channel up button 128 - pressed to increase the radio channel or television channel, as the case may be;
Channel down button 130 - pressed to decrease the radio channel or television channel, as the case may be;
Volume up button 132 - pressed to increase the radio volume or television volume, as the case may be; and
Volume down button 134 - pressed to decrease the radio volume of television volume, as the case may be.

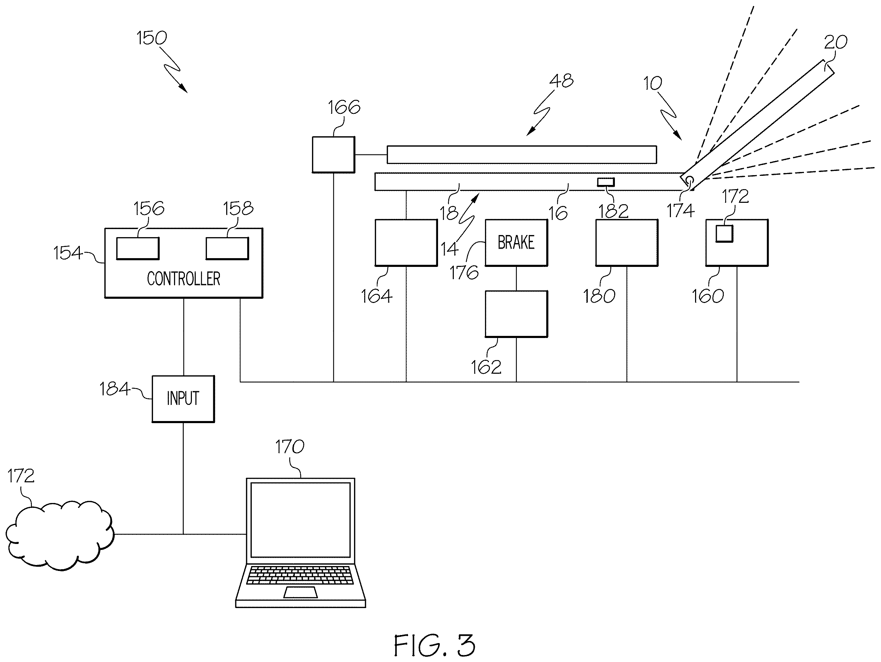

The above list of features are exemplary and not meant to limit the number and type of features of the bed 10. Any suitable number of the various features of the bed 10 may be controlled manually using one or more controls provided by either the subject control unit 70 or control pod 44. It should be noted that while the term "button" is used herein, any type of suitable actuatable device or area, such as part of the graphical user interface, that can be touched, pushed, slid, etc. to provide an input that can be used to control features of the bed 10. In some embodiments, artificial intelligence and/or voice assistants may be used to operate and/or control features of the bed, and/or to place one or more beds in compliance with the Falls Protocol. That is, the subject control unit 70 and/or the control pod 44 may include programming and/or functionality for various tasks including, but not limited to, receiving inputs such as voiced inputs, parsing the inputs, interfacing with other components (remote servers, etc.) to transmit and/or receive data corresponding to the inputs and/or responses from an artificial intelligence program, and providing an output to a user accordingly. As will be described in greater detail below, some or all of the various bed 10 control features may be controlled automatically and remotely using a bed control system 150 (FIG. 3) that is in communication with a bed controller 152 of the bed 10.

Referring to FIG. 3, the bed control system 150 includes the bed 10 that includes the bed controller 154 including a processor 156 that is configured to carry out instructions saved in memory 158. The processor 156 may include any device capable of executing machine-readable instructions stored on a non-transitory computing device-readable medium. The memory 158 is communicatively coupled to the processor 156 and may be configured as volatile and/or nonvolatile memory and, as such, may include random access memory (including SRAM, DRAM, and/or other types of RAM), flash memory, secure digital (SD) memory, registers, and/or other types of non-transitory computing device-readable mediums. Depending on the particular embodiment, these non-transitory computing device-readable mediums may reside within the bed 10 and/or external to the bed 10. The memory 158 may be configured to store one or more pieces of logic. The memory 158 may include one or more memory modules. The embodiments described herein may utilize a distributed computing arrangement to perform any portion of the logic described herein.

The memory 158 may include logic that, when carried out by processor 156, is configured to control operation of any one or more actuators 160, 162, 164 and 166. The actuators 160, 162, 164 and 166 are each configured to carry out an operation for adjusting a feature of the bed 10 that is related to the preset risk protocol associated with the bed 10. The preset risk protocol, such as the Falls Protocol may be entered and saved in a master computing device 170 that is communicatively coupled to the bed controller 154. For example, the master computing device 170 may be communicatively coupled directly to the bed controller 154 (e.g., through a wired or wireless connection) and/or may be communicatively coupled to the bed controller 154 through the internet 172. While a personal computer 170 is shown, the master computing device can be any suitable computing device, such as a handheld computing device like a tablet, smart phone with a web application, etc.

In the illustrated example, the actuators 160, 162, 164 and 166 may carry out functions related to Head of Bed Angle, Bed Braking, Bed Height and Side Rail Height. In particular, based on the Falls Protocol, the bed controller 154 instructs actuator 160 to move head section 20 to a predetermined angle to horizontal, such as 30 degrees. Raising the head section 20 can cause a subject to bend at the waist thereby making it more difficult to roll. The actuator 160 may be, for example, a hydraulic or pneumatic actuator, pump, motor or any other suitable actuator operatively coupled to the head section and configured to automatically raise and lower the head section 20. In some embodiments, a head-of-bed angle sensor 172 may provide input to the bed controller 154 of the current angle of the head section 20. While the angle sensor 172 is shown as part of the actuator 160, the angle sensor 172 may be located in or adjacent head section 20. The head section 20 is diagrammatically shown to pivot relative to the upper frame 16 about a pivot axis 174; however, in other embodiments the head section 20 can pivot about a moving axis, compound axis, or the like. Accordingly, all types of connections for coupling one deck section of a bed to another are within the scope of this disclosure including simple pivots, compound pivots, reduced-shear pivots, and pivots having arcuate tracks or slots, just to name a few.

Angle sensor 172 may be, for example, an accelerometer (single-axis or multi-axis) in some embodiments. In such embodiments, the HOB angle can be measured with respect to a horizontal reference axis and/or with respect to a vertical reference axis depending upon the orientation of the accelerometer relative to head section 20 and depending upon the type of accelerometer. In other embodiments, angle sensor 172 includes a rotary potentiometer which measures the HOB angle between head section 20 and another portion of frame 14 of the bed 10. In further embodiments, angle sensor 172 may be part of actuator 160 and can have an output that correlates to the HOB angle. Actuator 160 may include, for example, a shaft encoder, a Hall effect sensor, a rotary potentiometer, or some other sensor which serves as angle sensor of bed 10. Similar such sensors can include in other actuators or regions of the bed 10, in some embodiments, and can be used to determine the position of the bed such as the height and/or amount of tilt of one or more sections of the bed 10.

Similarly, based on the Falls Protocol, the bed controller 154 can instruct actuator 162 to actuate brake 176, which is configured to lock position of any one or more, such as two, of the casters 30. The actuator 162 may be, for example, a hydraulic or pneumatic actuator, motor or any other suitable actuator operatively coupled to the brake 176 and configured to automatically lock and unlock the brake 176. Locking the brake 176 can inhibit movement of the bed 10 when loading and unloading a subject on and off of the bed 10.

Based on the Falls Protocol, the bed controller 154 instructs actuator 164 to raise and/or lower the support deck 18 to a predetermined height. Again, the actuator 164 may be, for example, a hydraulic or pneumatic actuator, motor or any other suitable actuator operatively coupled to the support deck 18 and configured to automatically raise and lower the support deck 18. Lowering the support deck 18 can place the support deck 18 closer to the ground, which can reduce a fall risk when entering and/or exiting the bed 10.

Based on the Falls Protocol, the bed controller 154 instructs actuator 166 to raise and/or lower one, some or all of the side rails 48a, 48b, 48c, 48d. The actuator 166 may be, for example, a hydraulic or pneumatic actuator, motor or any other suitable actuator operatively coupled to one, some or all of the side rails 48a, 48b, 48c, 48d and configured to automatically raise and lower the one, some or all of the side rails 48a, 48b, 48c, 48d. Raising the side rails 48a, 48b, 48c, 48d can inhibit falling from the bed 10 and lowering the side rails 48a, 48b, 48c, 48d can assist in exiting the bed 10.

The bed controller 154 may also be communicatively coupled to a bed exit system 180. The bed controller 154 can activate the bed exit system 180 based on the Falls Protocol, which can provide an alarm to a caregiver based on subject position, exiting or out of bed condition occurs. In some embodiments, sensors, such as a camera, weight sensor, position sensor, etc., may be used to monitor position of the patient before any one or more of the changes described herein are made to the bed 10 in the interest of the bed occupant so that adverse actions are not taken that may harm or reduce comfort of the bed occupant or those in proximity to the bed 10. When a subject moves toward either of the side rails 48a, 48b, 48c, 48d or moves away from the head section 20, moves away from the center of the bed 10 toward an egress point or the subject's weight shifts significantly off the frame of the bed 10, an alarm can be provided to the caregiver. A sensor 182 may be provided in the bed 10 that can be used by the bed exit system 180 to check for the alarm conditions.

In this regard, according to the falls risk protocol example given herein, the bed exit system 180 can monitor a position of the subject relative to the base deck 14, support deck 18 can be required to be in a lowest position as sensed by sensors of actuators 164, side rails 48a, 48b, 48c, 48d, 60 of the frame 40 are required to be in the respective raised positions as sensed by side rail position sensors, also represented by element 166, and the caster brake of one or more of casters 30 is required to be braked or set as sensed by caster braking sensors, also represented by element 162. Thus, if the bed exit system 180 indicates that the subject is moving toward exiting bed 10 by a threshold amount (including exiting the bed 12) such movement beyond the threshold is considered to be a violation of the Falls Protocol assuming the falls protocol is enabled. Similarly, if any of side rails 48a, 48b, 48c, 48d are moved out of their raised positions, or if the one or more casters 30 are released or unbraked, each of those unwanted conditions is considered to be a violation of the Falls Protocol assuming the falls protocol is enabled.

A user input 184 may be communicatively coupled to the bed controller 154 that can be used by the caregiver to apply the Falls Protocol to the bed 10. As an example, the user input 184 may include a button that can be actuated by the caregiver, which sends a signal to the bed controller 154. The bed controller 154 can then send the current bed settings to the master computing device 170, which can check the current bed settings against the Falls Protocol entered into memory. To the extent that the current bed settings are different than the Falls Protocol, the master computing device 170 can then instruct the bed controller 154 to change the bed settings to be consistent with the Falls Protocol. All the bed settings can be changed to the Falls Protocol simultaneously and with the actuation of a single button upon a single, manual input event using the user input 184. The term "single, manual input event" refers to an actuation upon the user input 184 only once that results in multiple current bed settings being sent to the master computing device 170 for comparison with the preset risk protocol.

In some instances, if the subject needs to leave the room, for a procedure or other reason, the caregiver can set the room to Out of Room status. When Out of Room status is enabled, alerts can be paused and recurring reminders can be placed on hold automatically. Before the subject returns to the room, the caregiver can actuate the user input 184, which can again place the bed 10 in compliance with the Falls Protocol. When the subject is returned and is detected in the bed 10, the bed control system 150 can automatically restart reminders and the exit alarm can be armed.

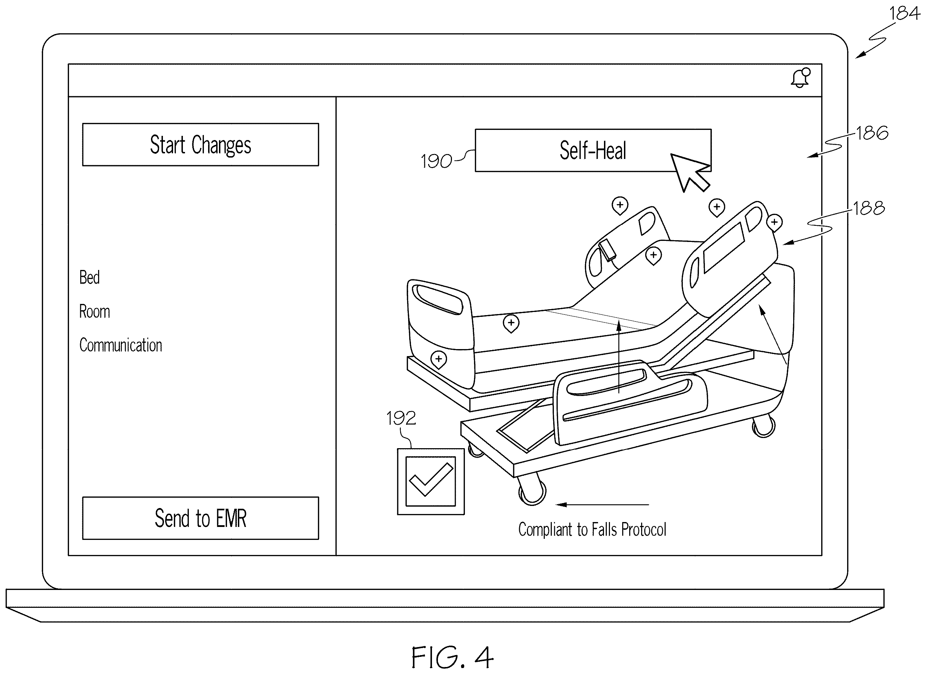

Referring to FIG. 4, the user input 184 may be, as an example, a computing device that includes a display 186. The display 186 may provide a digital representation 188 of the bed 10 in a current state. For example, the bed 10 may, in the current state, have all side rails 48a, 48b, 48c, 48d down and the HOB angle may be set at zero. When the caregiver selects the button 190, the bed controller 154 can apply self-heal, which automatically sets all bed parameters to the Falls Protocol, such as an HOB angle of 30 degrees, side rails 48a, 48b, 48c, 48d up, the bed elevation low, brake locked and bed exit alarm ON. When all bed parameters are set to the preset risk protocol, an indication 192 may be provided that the Falls Protocol conditions have been successfully set. If the current state of the bed 10 does not meet the Falls Protocol, then a different negative indication may be displayed.

In some embodiments, artificial intelligence (AI) and voice assistants may be used to control features of the bed and to place one or more beds in compliance with the Falls Protocol. For example, "Scotty," a voice-activated assistant technology by Baxter International may be used to interface with a caregiver and generate an AI query to determine the status of a certain bed, multiple beds or all of the beds to determine compliance with the Falls Protocol and then bring the beds back into compliance or otherwise some other bed setting using the master computing device. It should be appreciated that other hardware components used for hosting and/or operating AI and voice assistant software, whether integrated into the components described herein and/or communicatively coupled to components described herein are contemplated and included in the scope of the present disclosure.

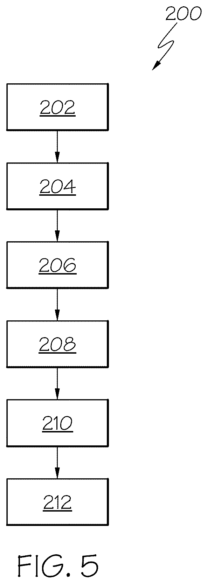

Referring now to FIG. 5, a method 200 of applying a Falls Protocol to a bed 10 includes, at step 202, entering the Falls Protocol details into the bed control system 150 using the master computing device 170. As described above, the Falls Protocol can include bed parameters, such as Head of Bed Angle (e.g., 30 degrees from horizontal), Bed Brake Locked, Bed Height Low, Bedrails Up and Bed Exit On. The Falls Protocol details may be provided from another database or can even be manually entered by a caregiver. At step 204, a subject may be placed on the bed 10. Once the subject is in position, the user input 184 may be actuated by the caregiver at step 206. At step 208, the current bed settings may be sent to the master computing device 170 and the master computing device 170 can compare the current bed settings against the Falls Protocol saved in memory at step 210. At step 212, the master computing device 170 can send instructions to the bed controller 154 as to which bed parameters should be adjusted based on the comparison between the current bed settings and the Falls Protocol. The bed controller 154 can then instruct the actuators 160, 162, 164, 166 and the bed exit system 180 accordingly.

A call system, such as the NAVICARE^{®} Nurse Call (NNC) system available from Baxter International, Inc. can be part of the bed control system 150 and allow caregivers control various bed settings remotely from the beds 10 and be used to monitor bed status data to see if reminders, alarms or other alert conditions have been triggered. If so, the call system can generate an alert for the caregiver. Alert notifications can include, for example, sending a message to a wireless communication device (e.g., pager, smart phone, tablet computer, telephone handset, etc.) carried by an assigned caregiver or lighting up a light situated outside the subject room having the bed with the alert condition.

As Falls Protocols are discussed primarily above as they may be used to control a number of bed 10 parameters, as mentioned above, the bed control system 150 may be used to control one or more of the bed parameters, such as one or more of those discussed above, based on other protocol types, such as a Pulmonary Protocol and a Skin Protocol. According to a Pulmonary Protocol example, head section 20 of the subject support deck 18 may be elevated above a threshold angle as measured by angle sensor 172. The threshold angle may be about 30 degrees, for example. In some embodiments, user input may be used to adjust the threshold angle to an angle greater than 30 degrees (e.g., 45 degrees or 60 degrees) or to an angle less than 30 degrees (e.g., 17 degrees or 10 degrees). Thus, if the HOB angle falls below the threshold angle, that may be considered to be a violation of the pulmonary protocol, assuming the pulmonary protocol is enabled. Having the HOB angle above a threshold angle, such as 30 degrees, helps to prevent ventilator assisted pneumonia (VAP) in some subjects. Alternatively or additionally, the pulmonary protocol may require that the subject undergo certain pulmonary therapies performed by the bed 10 within certain time intervals. Examples of such pulmonary therapies include, for example, continuous lateral rotation therapy (CLRT) using rotation bladders of mattress 28 and/or percussion/vibration (P&V) therapy using P&V bladders of mattress 28. Thus, CLRT or P&V therapy may be required for ½ hour, 1 hour, 2 hours, etc. twice per day or at least once within in any 8 hour nursing shift according to the pulmonary protocol, just to give a couple of nonlimiting examples.

According to the skin protocol example given herein, the subject may be required to move relative to the frame 14 by a threshold amount so as not to be stationary for a threshold amount of time. Such movement may be detected by the bed control system 150. Alternatively or additionally, the skin protocol may require the operation of certain features of mattress 28 for certain periods of time or at certain intervals. For example, an alternating pressure feature of mattress 28 may be required to be operating substantially continuously while the subject is in bed 10. Alternating pressure refers to sequentially inflating and deflating every other laterally extending bladder of two sets of interleaved or interdigitated bladders of mattress 28. Alternatively or additionally, the skin protocol may require a microclimate management layer or low air loss topper of mattress 28 to have air circulated therethrough to remove or reduce the amount of moisture and/or heat between the subject and the upper surface of mattress 28. Further alternatively or additionally, the skin protocol may require that turn assist bladders of the mattress 28 be used from time to time to turn the subject to their left side and to their right side. When the safe skin protocol is enabled, the bed control system 150 can monitor the various time thresholds for subject movement, alternating pressure, microclimate management operation, and turn assist, as the case may be.

The above-described beds and bed control systems can allow caregivers to set the beds with the current Falls Protocol or other preset risk protocol of a particular hospital in a reliable manner. The Falls Protocol can be provided to the bed control system and a user control can be used to adjust the current bed settings to be in compliance with the Falls Protocol. Further, the Falls Protocol parameters can be adjusted automatically in case the caregiver is unsure of the Falls Protocol or the Falls Protocol is changed and simultaneously, which can eliminate the need to separately adjust each parameter in a sequential fashion, which can be time-consuming.

Embodiments can be described with reference to the following numbered clauses, with preferred features laid out in the dependent clauses:
Clause 1: A bed control system for controlling settings of a bed, the bed control system comprising: a bed controller that is configured to control multiple actuators of the bed, the actuators configured to move the bed based on instructions from the bed controller; and a master computing device remote from the bed and communicatively coupled to the bed controller, the master computing device comprising a memory storing a preset risk protocol and logic that, when implemented by a processor, sends adjustment instructions to the bed controller based on a comparison between multiple current bed settings of the bed and the preset risk protocol.
Clause 2: The bed control system of clause 1, wherein the bed controller is configured to send the multiple current bed settings to the master computing device for the comparison based on a single, manual input event to a user input.
Clause 3: The bed control system of clause 1 or 2, wherein the actuators comprise a first actuator that is configured to adjust an angle of a head section of the bed.
Clause 4: The bed control system of clause 3, wherein the actuators comprise a second actuator that is configured to lock a brake configured to inhibit movement of the bed.
Clause 5: The bed control system of clause 4, wherein the actuators comprise a third actuator that is configured to raise and lower a deck of the bed.
Clause 6: The bed control system of clause 5, wherein the actuators comprise a fourth actuator configured to raise and lower a side rail of the bed.
Clause 7: The bed control system of clause 6, wherein the bed controller is configured to activate a bed exit system.
Clause 8: The bed control system of clause 6 or 7, wherein the bed controller is configured to operate the first, second, third and fourth actuators simultaneously based on the adjustment instructions from the master computing device.
Clause 9: The bed control system of any of the above clauses, wherein the bed comprises a bed exit system comprising an alarm configured to provide an indication to a caregiver based on a position of a subject on the bed.
Clause 10: The bed control system of clause 9, wherein the bed controller is configured to operate the bed exit system based on the adjustment instructions from the master computing device.
Clause 11: The bed control system of any of claims 1-10, wherein the master computing device is configured to be controlled by a voice-activated artificial intelligence assistant.
Clause 12: A method for controlling settings of a bed using a bed control system, the method comprising: sending current bed settings of the bed to a master computing device that is remote from the bed using a bed controller of the bed, the bed controller configured to control multiple actuators of the bed, the actuators configured to move the bed based on adjustment instructions from the bed controller; and comparing the current bed settings to a preset risk protocol saved in memory using the master computing device and sending adjustment instructions from the master computing device to the bed controller based on the comparison thereby causing the bed controller to adjust the multiple actuators of the bed.
Clause 13: The method of clause 12, wherein sending current bed settings of the bed to a master computing device that is remote from the bed using the bed controller occurs upon a single, manual input event to a user input.
Clause 14: The method of clause 12 or 13, wherein the actuators comprise a first actuator that is configured to adjust an angle of a head section of the bed.
Clause 15: The method of clause 14, wherein the actuators comprise a second actuator that is configured to lock a brake configured to inhibit movement of the bed.
Clause 16: The method of clause 15, wherein the actuators comprise a third actuator that is configured to raise and lower a deck of the bed.
Clause 17: The method of clause 16, wherein the actuators comprise a fourth actuator configured to raise and lower a side rail of the bed.
Clause 18: The method of clause 17, wherein the bed controller is configured to activate a bed exit system.
Clause 19: The method of clause 17, wherein the bed controller operating the first, second, third and fourth actuators simultaneously based on the adjustment instructions from the master computing device.
Clause 20: The method of clause 17, wherein the bed comprises a bed exit system comprising an alarm configured to provide an indication to a caregiver based on a position of a subject on the bed.
Clause 21: The method of clause 20, wherein the bed controller operating the bed exit system based on the adjustment instructions from the master computing device.
Clause 22: The method of any one of clauses 12-21 further comprising entering the preset risk protocol into memory of the master computing device.
Clause 23: The method of any of claims 11-22 further comprising controlling the master computing device by a voice-activated artificial intelligence assistant.
Clause 24: A method for controlling settings of a bed using a bed control system, the method comprising: sending current bed settings of the bed to a master computing device that is remote from the bed using a bed controller of the bed, the bed controller configured to control Head of Bed Angle, Bed Braking, Bed Height, Bed Rails Position and Bed Exit On/Off based on adjustment instructions from the master computing device; and comparing current bed settings to a Falls Protocol using the master computing device and sending adjustment instructions to the bed controller based on the comparison thereby causing the bed controller to adjust the bed based on the comparison.
Clause 25: The method of clause 24, wherein sending current bed settings of the bed to the master computing device that is remote from the bed using the bed controller of the bed occurs upon a single, manual input event to a user input
Clause 26: The method of clause 24 or 25, wherein the bed controller is configured to adjust Head of Bed Angle, Bed Braking, Bed Height, Bed Rails Position and Bed Exit On/Off based on instructions from the bed controller simultaneously.
Clause 27: The method of any of clauses 24-26, wherein the bed controller is configured to control multiple actuators of the bed, the actuators comprise a first actuator that is configured to adjust an angle of a head section of the bed.
Clause 28: The method of clause 27, wherein the actuators comprise a second actuator that is configured to lock a brake configured to inhibit movement of the bed.
Clause 29: The method of clause 28, wherein the actuators comprise a third actuator that is configured to raise and lower a deck of the bed.
Clause 30: The method of clause 29, wherein the actuators comprise a fourth actuator configured to raise and lower a side rail of the bed.
Clause 31: The method of clause 30, wherein the bed controller is configured to activate a bed exit system.
Clause 32: The method of clause 30, wherein the bed controller operating the first, second, third and fourth actuators simultaneously based on the adjustment instructions from the master computing device.
Clause 33: The method of any of clauses 30-32, wherein the bed comprises a bed exit system comprising an alarm configured to provide an indication to a caregiver based on a position of a subject on the bed.
Clause 34: The method of clause 33, wherein the bed controller operating the bed exit system based on the adjustment instructions from the master computing device.
Clause 35: The method of any of clauses 24-34 further comprising entering the Falls Protocol into memory of the master computing device.

Based on the foregoing, it should be understood that the person lifting apparatuses described herein provide multiple lifting straps along with a controller that monitors and compares current draws from their associated lifting strap feeding devices to detect an imbalance condition.

It will be apparent to those skilled in the art that various modifications and variations can be made to the embodiments described herein without departing from the spirit and scope of the claimed subject matter. Thus it is intended that the specification cover the modifications and variations of the various embodiments described herein provided such modification and variations come within the scope of the appended claims and their equivalents.

## Claims

1. A bed control system for controlling settings of a bed, the bed control system comprising:
a bed controller that is configured to control multiple actuators of the bed, the actuators configured to move the bed based on instructions from the bed controller; and
a master computing device remote from the bed and communicatively coupled to the bed controller, the master computing device comprising a memory storing a preset risk protocol and logic that, when implemented by a processor, sends adjustment instructions to the bed controller based on a comparison between multiple current bed settings of the bed and the preset risk protocol.

2. The bed control system of claim 1, wherein the bed controller is configured to send the multiple current bed settings to the master computing device for the comparison based on a single, manual input event to a user input.

3. The bed control system of claim 1 or 2, wherein the actuators comprise a first actuator that is configured to adjust an angle of a head section of the bed and optionally, wherein the actuators comprise a second actuator that is configured to lock a brake configured to inhibit movement of the bed, and optionally, wherein the actuators comprise a third actuator that is configured to raise and lower a deck of the bed and optionally, wherein the actuators comprise a fourth actuator configured to raise and lower a side rail of the bed.

4. The bed control system of claim 3, wherein the bed controller is configured to activate a bed exit system.

5. The bed control system of claim 3 or 4, wherein the bed controller is configured to operate the first, second, third and fourth actuators simultaneously based on the adjustment instructions from the master computing device.

6. The bed control system of any of the above claims, wherein the bed comprises a bed exit system comprising an alarm configured to provide an indication to a caregiver based on a position of a subject on the bed.

7. The bed control system of claim 6, wherein the bed controller is configured to operate the bed exit system based on the adjustment instructions from the master computing device.

8. The bed control system of any of the above claims, wherein the master computing device is configured to be controlled by a voice-activated artificial intelligence assistant.

9. A method for controlling settings of a bed using a bed control system, the method comprising:
sending current bed settings of the bed to a master computing device that is remote from the bed using a bed controller of the bed, the bed controller configured to control multiple actuators of the bed, the actuators configured to move the bed based on adjustment instructions from the bed controller; and
comparing the current bed settings to a preset risk protocol saved in memory using the master computing device and sending adjustment instructions from the master computing device to the bed controller based on the comparison thereby causing the bed controller to adjust the multiple actuators of the bed.

10. The method of claim 9, wherein sending current bed settings of the bed to a master computing device that is remote from the bed using the bed controller occurs upon a single, manual input event to a user input.

11. The method of claim 9 or 10, wherein the actuators comprise a first actuator that is configured to adjust an angle of a head section of the bed and optionally, wherein the actuators comprise a second actuator that is configured to lock a brake configured to inhibit movement of the bed and optionally, wherein the actuators comprise a third actuator that is configured to raise and lower a deck of the bed and optionally, wherein the actuators comprise a fourth actuator configured to raise and lower a side rail of the bed.

12. The method of claim 11, wherein the bed controller is configured to activate a bed exit system.

13. The method of claim 11, wherein the bed controller operating the first, second, third and fourth actuators simultaneously based on the adjustment instructions from the master computing device.

14. The method of claim 11, wherein the bed comprises a bed exit system comprising an alarm configured to provide an indication to a caregiver based on a position of a subject on the bed.

15. The method of claim 14, wherein the bed controller operating the bed exit system based on the adjustment instructions from the master computing device.
